(19) Europäisches Patentamt
European Patent Office
Office européen des brevets

(11) Veröffentlichungsnummer: **0 245 785**
**A2**

# (12) EUROPÄISCHE PATENTANMELDUNG

(21) Anmeldenummer: **87106653.6**

(22) Anmeldetag: **07.05.87**

(51) Int. Cl.⁴: **C07D 231/44**, C07D 403/04, A01N 43/56, A01N 43/36

(30) Priorität: **16.05.86 DE 3616681**

(43) Veröffentlichungstag der Anmeldung: **19.11.87 Patentblatt 87/47**

(84) Benannte Vertragsstaaten: **AT BE CH DE ES FR GB GR IT LI NL**

(71) Anmelder: **BAYER AG**
**Konzernverwaltung RP Patentabteilung**
**D-5090 Leverkusen 1 Bayerwerk(DE)**

(72) Erfinder: **Lindig, Markus, Dr.**
**Dahlienweg 16**
**D-4010 Hilden(DE)**
Erfinder: **Becker, Benedikt, Dr.**
**Metzkausener Strasse 14**
**D-4020 Mettmann(DE)**

## (54) 1-Aralkylpyrazole.

(57) Es werden neue 1-Aralkylpyrazole der Formel (I)

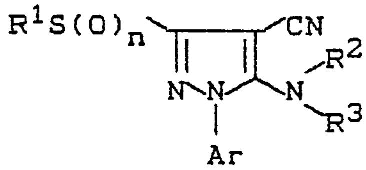

(I)

bereitgestellt, in welcher
Ar für gegebenenfalls substituiertes Aralkyl steht,
$R^1$ für Alkyl steht,
$R^2$ für Wasserstoff, Alkyl, Halogenalkyl, gegebenenfalls substituiertes Benzyl oder für die Reste $-COR^4$, $-COOR^4$, $-NR^5R^6$, $-CONR^5R^6$ steht,
wobei
$R^4$ für Wasserstoff, Alkyl, Halogenalkyl, Alkoxyalkyl und
$R^5$ und $R^6$ für Wasserstoff und/oder Alkyl steht,
$R^3$ für Wasserstoff oder Alkyl steht oder
$R^2$ und $R^3$ gemeinsam für die Gruppierungen $-CO-(CH_2)_m-$, $-(CH_2)_{\overline{n}}$ oder $-CH=CH-CH=CH-$stehen, wobei
m für die Zahlen 2, 3, 4, oder 5 steht und
n für die Zahlen 0, 1 oder 2 steht.

Die neuen Aralkylpyrazole (I) können nach verschiedenen im Anmeldungstext beschriebenen Verfahren hergestellt werden und besitzen ausgezeichnete akarizide Wirksamkeit.

EP 0 245 785 A2

# 1-Aralkylpyrazole

Die vorliegende Erfindung betrifft neue 1-Aralkylpyrazole, mehrere Verfahren zu ihrer Herstellung, ihre Verwendung als Schädlingsbekämpfungsmitel, insbesondere als Akarizide sowie neue Zwischenprodukte zu ihrer Herstellung und Verfahren zu deren Herstellung.

Es ist bereits bekannt, daß Pyrazolderivate, wie beispielsweise das 1-Cyclohexyl-5-[N,N-(dimethyl)-carbamoyloxy]-3-methylthiomethyl-pyrazol, das 1-Cyclohexyl-5-[N,N-(dimethyl)-carbamoyloxy]-3-methylsulfinylmethyl-pyrazol oder das 1-Cyclohexyl-5-[N,N-(dimethyl)-carbamoyloxy]-3-methylsulfonylmethyl-pyrazol akarizide Eigenschaften besitzen (vgl. z.B. DE-OS 28 39 270).

Die Wirkungshöhe bzw. die Wirkungsdauer dieser Verbindungen sind jedoch insbesondere bei bestimmten Spinnenmilbenarten oder bei niedrigen Anwendungskonzentrationen nicht immer völlig zufriedenstellend.

Es wurden neue 1-Aralkylpyrazole der Formel (I)

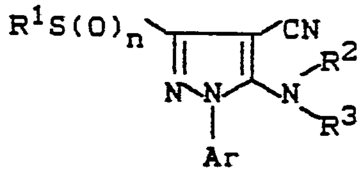

(I)

gefunden, in welcher

Ar für gegebenenfalls substituiertes Aralkyl steht,

$R^1$ für Alkyl steht,

$R^2$ für Wasserstoff, Alkyl, Halogenalkyl, gegebenenfalls substituiertes Benzyl oder für die Reste $-COR^4$, $-COOR^4$, $-NR^5R^6$, $-CONR^5R^6$ steht,

wobei

$R^4$ für Wasserstoff, Alkyl, Halogenalkyl, Alkoxyalkyl und

$R^5$ und $R^6$ für Wasserstoff und/oder Alkyl steht,

$R^3$ für Wasserstoff oder Alkyl steht oder

$R^2$ und $R^3$ gemeinsam für die Gruppierungen $-CO-(CH_2)_m-$, $-(CH_2)_{\overline{m}}$ oder $-CH=CH-CH=CH-$stehen, wobei

m für die Zahlen 2, 3, 4 oder 5 steht und

n für die Zahlen 0, 1 oder 2 steht.

Man erhält 1-Aralkylpyrazole der Formel (I)

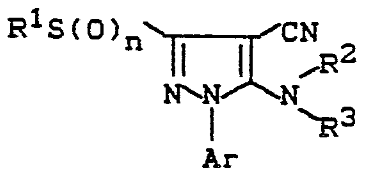

(I)

in welcher

Ar für gegebenenfalls substituiertes Aralkyl steht,

$R^1$ für Alkyl steht,

$R^2$ für Wasserstoff, Alkyl, Halogenalkyl, gegebenenfalls substituiertes Benzyl oder für die Reste $-COR^4$, $-COOR^4$, $-NR^5R^6$, $-CONR^5R^6$ steht,

wobei

$R^4$ für Wasserstoff, Alkyl, Halogenalkyl, Alkoxyalkyl und

$R^5$ und $R^6$ für Wasserstoff und/oder Alkyl steht,

$R^3$ für Wasserstoff oder Alkyl steht oder

$R^2$ und $R^3$ gemeinsam für die Gruppierungen $-CO-(CH_2)_m-$, $-(CH_2)_m-$oder $-CH=CH-CH=CH-$stehen, wobei

m für die Zahlen 2, 3, 4 oder 5 steht und

n für die Zahlen 0, 1 oder 2 steht,

wenn man

(a) zum Erhalt von Verbindungen der Formel (Ia)

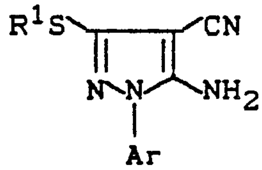

(Ia)

in welcher
$R^1$ und Ar die unter Formel (I) gegebene Bedeutung besitzen,
Aralkylhydrazine der Formel (II)
Ar-NH-$NH_2$ (II)
in welcher
Ar die oben angegebene Bedeutung besitzt
oder deren Säureadditionssalze mit 1,1-Dicyanethylen-Derivaten der Formel (III)

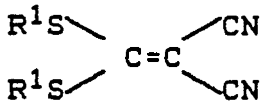

(III)

in welcher
$R^1$ die oben angegebene Bedeutung besitzt,
gegebenenfalls in Gegenwart eines Lösungsmittels umsetzt,
oder wenn man

(b) zum Erhalt von Verbindungen der Formel (Ib)

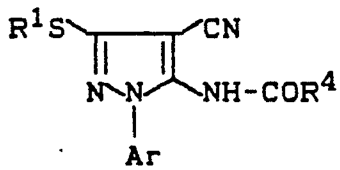

(Ib)

in welcher
Ar, $R^1$ und $R^4$ die unter Formel (I) angegebene Bedeutung besitzen,
1-Arylalkyl-4-cyan-5-amino-pyrazole der Formel (Ia)

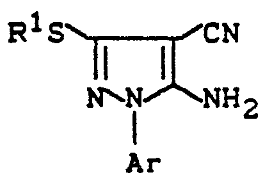

(Ia)

in welcher
Ar und $R^1$ die oben angegebene Bedeutung besitzen
mit Verbindungen der Formel

$$X\text{-}CO\text{-}R^4 \qquad (IV)$$

wobei
X = aktivierte Abgangsgruppe (wie Alkoxy, OH, Halogen) umsetzt,
oder wenn man

(c) zum Erhalt von Verbindungen der Formel (Ic)

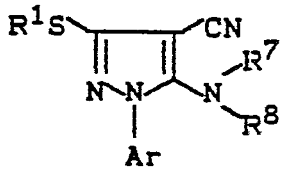

(Ic)

worin
Ar, $R^1$ die oben angegebene Bedeutung haben,
$R^7$ für Wasserstoff oder $-COR^4$ steht und
$R^8$ für Alkyl steht,
Verbindungen der Formel (V)

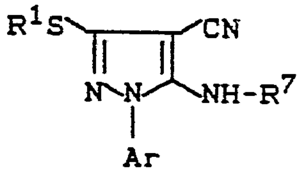

(V)

in welcher
Ar, $R^1$ und $R^7$ die oben angegebene Bedeutung besitzen,
mit Dialkylsulfat umsetzt,
oder wenn man
(d) zum Erhalt von Verbindungen der Formel (Id)

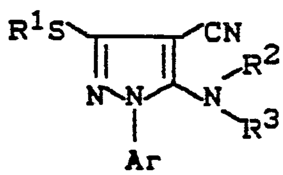

(Id)

in welcher
Ar, $R^1$, $R^2$ und $R^3$ die unter Formel (I) angegebene Bedeutung besitzen,
1-Arylalkyl-4-cyan-5-brom-pyrazole der Formel

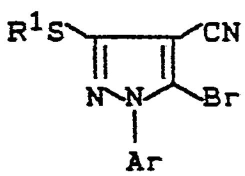

(VI)

in welcher
Ar und $R^1$ die oben angegebene Bedeutung besitzen,
mit Aminen der Formel

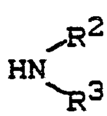

(VII)

umsetzt,
oder wenn man
(e) zum Erhalt von Verbindungen der Formel (Ie)

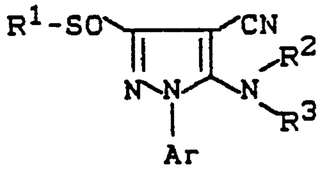

(Ie)

in welcher
Ar, $R^1$, $R^2$ und $R^3$ die oben angegebene Bedeutung besitzen,
1-Arylalkyl-4-cyan-pyrazole der Formel (Id)

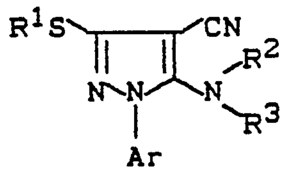

(Id)

in welcher
Ar, $R^1$, $R^2$ und $R^3$ die oben angegebene Bedeutung haben,
mit äquimolaren Mengen eines Oxidationsmittels umsetzt,
oder wenn man

(f) zum Erhalt von Verbindungen der Formel (If)

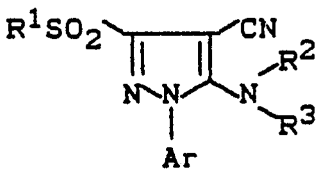

(If)

in welcher
Ar, $R^1$, $R^2$ und $R^3$ die oben angegebene Bedeutung haben,
1-Arylalkyl-4-cyan-pyrazole der Formel (Id)

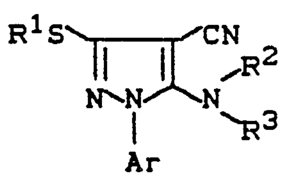

(Id)

mit mindestens der zweifach molaren Menge eines Oxidationsmittels umsetzt.

Schließlich wurde gefunden, daß die neuen 1-Aralkylpyrazole der allgemeinen Formel (I) pestizide und insbesondere akarizide Eigenschaften besitzen.

Überraschenderweise zeigen die erfindungsgemäßen 1-Aralkylpyrazole der allgemeinen Formel (I) eine erheblich bessere akarizide Wirksamkeit als die aus dem Stand der Technik bekannten Pyrazol-Derivate, wie beispielsweise die Verbindungen 1-Cyclohexyl-5-[N,N-(dimethyl)-carbamoyloxy]-3-methylthiomethyl-pyrazol, 1-Cyclohexyl-5-[N,N-(dimethyl)-carbamoyloxy]-3-methylsulfinylmethyl-pyrazol und 1-Cyclohexyl-5-[N,N-(dimethyl)-carbamoyloxy]-3-methylsulfonylmethyl-pyrazol, welche chemisch naheliegende Verbindungen sind.

Die erfindungsgemäßen 1-Aralkypyrazole sind durch die Formel (I) allgemein definiert. Bevorzugt sind Verbindungen der Formel (I), bei welchen
Ar für gegebenenfalls einfach bis dreifach, gleich oder verschieden substituiertes Phenylalkyl mit 1 bis 4 Kohlenstoffatomen im geradkettigen oder verzweigten Alkylteil steht, wobei als Substituenten des Phenylrestes genannt seien: Halogen, Halogenalkyl oder Halogenalkoxy mit jeweils vorzugsweise 1 oder 2 Kohlenstoffatomen und 1 bis 5 gleichen oder verschiedenen Halogenatomen (insbesondere Fluor oder Chlor) steht,
$R^1$ für Alkyl mit 1 bis 8 Kohlenstoffatomen steht,

$R^2$ für Wasserstoff, $C_1$-$C_8$-Alkyl, Halogenalkyl mit 1 oder 2 Kohlenstoffatomen und 1 bis 5 gleichen oder verschiedenen Halogenatomen (insbesondere Fluor oder Chlor) oder für gegebenenfalls einfach bis dreifach gleich oder verschieden substituiertes Benzyl steht, wobei als Phenylsubstituenten vorzugsweise genannt seien: Halogen, Alkyl mit 1 bis 4 Kohlenstoffatomen und Halogenalkyl mit 1 bis 2 Kohlenstoffatomen und 1 bis 5 gleichen oder verschiedenen Halogenatomen, sowie für die Reste $-COR^4$, $-COOR^4$, $-NR^5R^6$, $-CONR^5R^6$ steht, wobei

$R^4$ für Wasserstoff, $C_1$-$C_8$-Alkyl, Halogenalkyl mit 1 oder 2 Kohlenstoffatomen und 1 bis 5 gleichen oder verschiedenen Halogenatomen (insbesondere Fluor oder Chlor) oder Alkoxyalkyl mit 1 bis 4 Kohlenstoffatomen in jedem Alkylteil steht

und wobei

$R^5$ und $R^6$ für Wasserstoff oder $C_1$-$C_4$-Alkyl stehen,

$R^3$ für Wasserstoff oder $C_1$-$C_8$-Alkyl steht, oder

$R^2$ und $R^3$ gemeinsam für die Gruppierungen $-CO-(CH_2)_m-$, $-(CH_2)_m-$oder $-CH=CH-CH=CH-$stehen, wobei m für die Zahlen 2, 3, 4 oder 5 steht und

n für die Zahlen 0, 1 oder 2 steht.

Besonders bevorzugt sind 1-Aralkylpyrazole der Formel (I), in welcher

Ar für jeweils gegebenenfalls einfach oder zweifach, gleich oder verschieden durch Chlor oder Trifluormethyl substituiertes Benzyl, 1-Phenylethyl oder 2-Phenylethyl steht,

$R^1$ für Alkyl mit 1 bis 4 Kohlenstoffatomen steht,

$R^2$ für Wasserstoff, $C_1$-$C_4$-Alkyl oder für gegebenenfalls einfach oder zweifach, gleich oder verschieden substituiertes Benzyl steht, wobei als Phenylsubstituenten genannt seien: Fluor, Chlor, Brom, Methyl, Ethyl, Isopropyl und Trifluormethyl; sowie für die Reste $-NR^5R^6$, $-COR^4$, $-COOR^4$ und $-CONR^5R^6$ steht, wobei

$R^4$ für Wasserstoff, $C_1$-$C_4$-Alkyl oder Alkoxyalkyl mit 1 bis 2 Kohlenstoffatomen in jedem Alkylteil steht und

$R^5$ und $R^6$ für Wasserstoff oder $C_1$-$C_4$-Alkyl stehen,

$R^3$ für Wasserstoff oder $C_1$-$C_4$-Alkyl steht oder

$R^2$ und $R^3$ gemeinsam für die Gruppierungen $-CO-(CH_2)_m-$, $-(CH_2)_m-$oder $-CH=CH-CH=CH-$stehen, wobei m für die Zahlen 2, 3, 4 oder 5 steht und

n für die Zahlen 0, 1 oder 2 steht.

Im einzelnen seien außer den bei den Herstellungsbeispielen genannten Verbindungen die folgenden 1-Aralkylpyrazole der allgemeinen Formel (I) genannt:

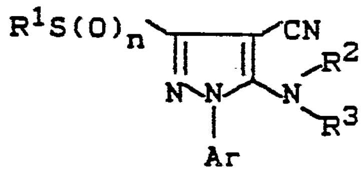

(I)

| Ar | $R^1$ | $R^2$ | $R^3$ | n |
|---|---|---|---|---|
| 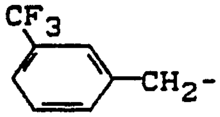 | $CH_3$ | $-NH_2$ | H | 0 |
| " | $CH_3$ | $-NH_2$ | $CH_3$ | 0 |
| " | $CH_3$ | $-COC_3H_7$-i | $CH_3$ | 0 |
| " | $CH_3$ | $-COCH_2C_3H_7$-i | $CH_3$ | 0 |
| " | $CH_3$ | $-COC_3H_7$-i | $CH_3$ | 2 |
| " | $CH_3$ | -CH=CH-CH=CH- | | 1 |
| " | $CH_3$ | $-CO-(CH_2)_3-$ | | 2 |
| " | $CH_3$ | $-COOCH_3$ | $CH_3$ | 0 |
| " | $CH_3$ | $-COOC_2H_5$ | $CH_3$ | 2 |
| " | $CH_3$ | $-CON(CH_3)_2$ | $CH_3$ | 0 |
| " | $CH_3$ | $-CH_2CH_2Cl$ | $CH_3$ | 1 |
| " | $CH_3$ | $-CO-(CH_2)_2-$ | | 0 |
| 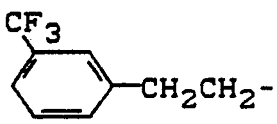 | $CH_3$ | H | H | 0 |
| " | $C_2H_5$ | H | H | 0 |
| 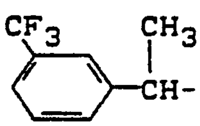 | $CH_3$ | H | H | 0 |

Verwendet man beispielsweise 3-Trifluormethyl-benzylhydrazin und 1,1-Dicyano-2,2-bis-(methylthio)-ethylen als Ausgangsstoffe, so läßt sich der Reaktionsablauf des erfindungsgemäßen Verfahrens (a) durch das folgende Schema darstellen:

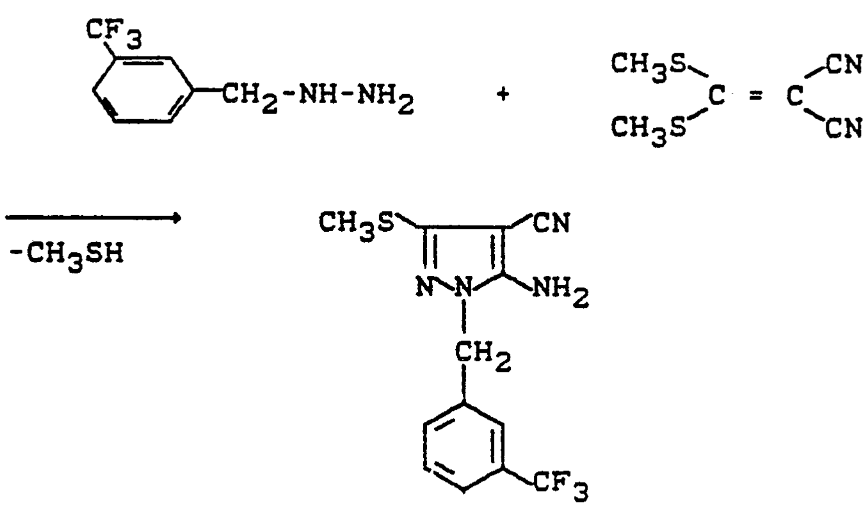

Verwendet man beispielsweise 1-(3-Trifluormethylbenzyl)3-methylmercapto-4-cyan-5-amino-pyrazol und Propionsäurechlorid als Ausgangsstoffe, so läßt sich der Reaktionsablauf des erfindungsgemäßen Verfahrens (b) durch das folgende Schema darstellen:

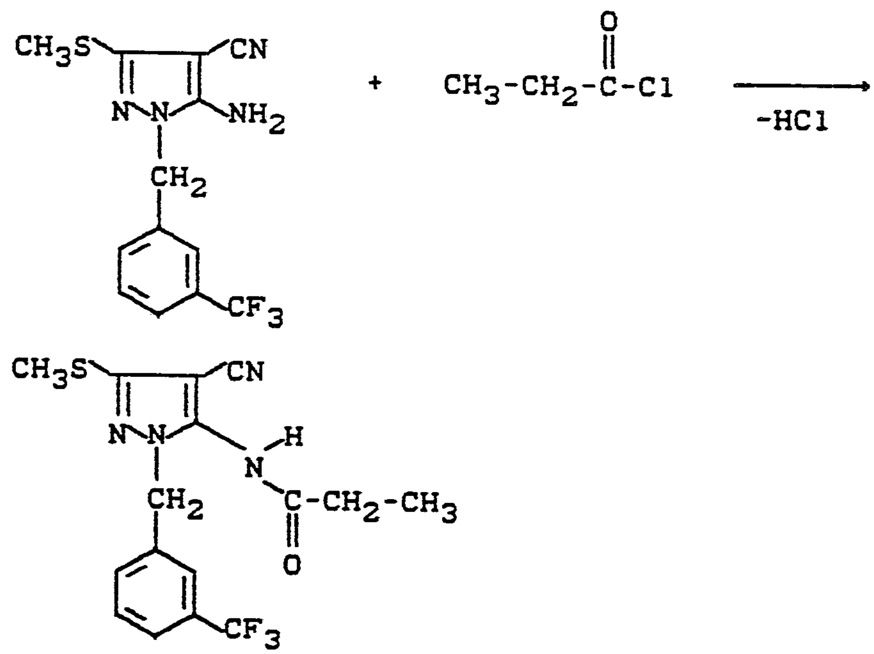


Verwendet man beispielsweise 1-(3-Trifluormethylbenzyl)-3-methylmercapto-4-cyan-5-propionylamino-pyrazol und Dimethylsulfat als Ausgangsstoffe, so läßt sich der Reaktionsablauf des erfindungsgemäßen Verfahrens (c) durch das folgende Schema darstellen:

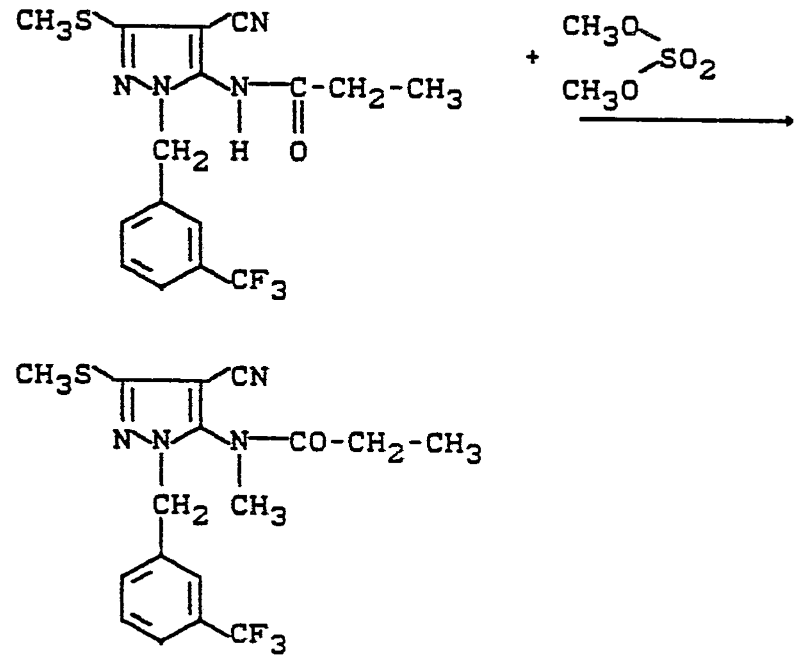

Verwendet man beispielsweise 1-(4-Trifluormethylbenzyl)-3-ethylmercapto-4-cyan-5-brom-pyrazol und Dimethylamin als Ausgangsstoffe, so läßt sich der Reaktionsablauf des erfindungsgemäßen Verfahrens (d) durch das folgende Schema darstellen:

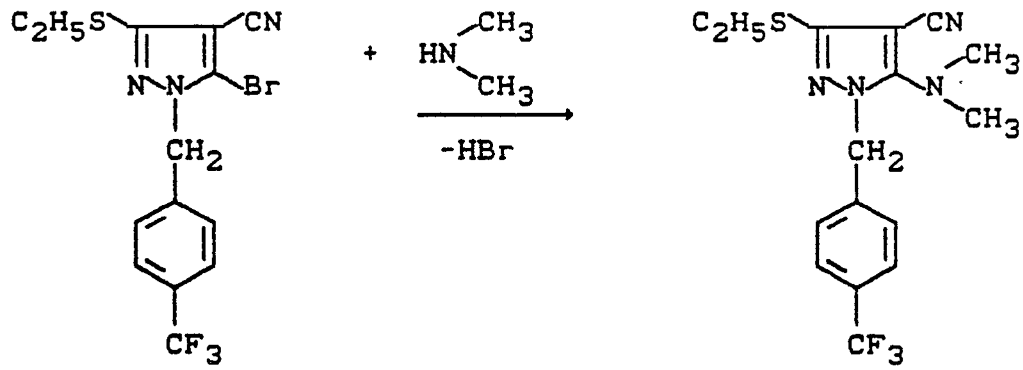


Verwendet man beispielsweise 1-(3-Trifluormethylbenzyl)-3-methylmercapto-4-cyan-5-amino-pyrazol und eine äquimolare Menge an 3-Chlor-perbenzoesäure als Ausgangsstoffe, so kann der Reaktionsablauf des erfindungsgemäßen Verfahrens (e) wie folgt dargestellt werden:

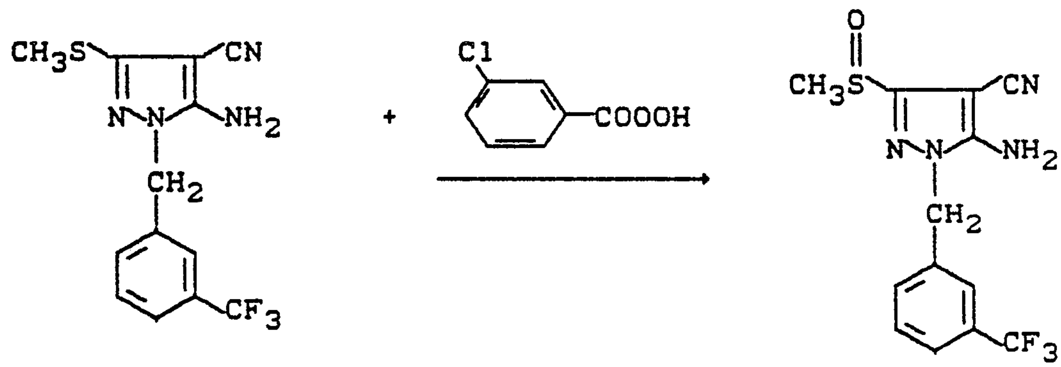

Verwendet man beispielsweise 1-(4-Trifluormethylphenylethyl)-3-methylmercapto-4-cyan-5-dimethylamino-pyrazol und die doppelt-molare Menge an 3-Chlor-perbenzoesäure als Ausgangsstoffe, so kann der Reaktionsablauf des erfindungsgemäßen Verfahrens (f) wie folgt dargestellt werden:

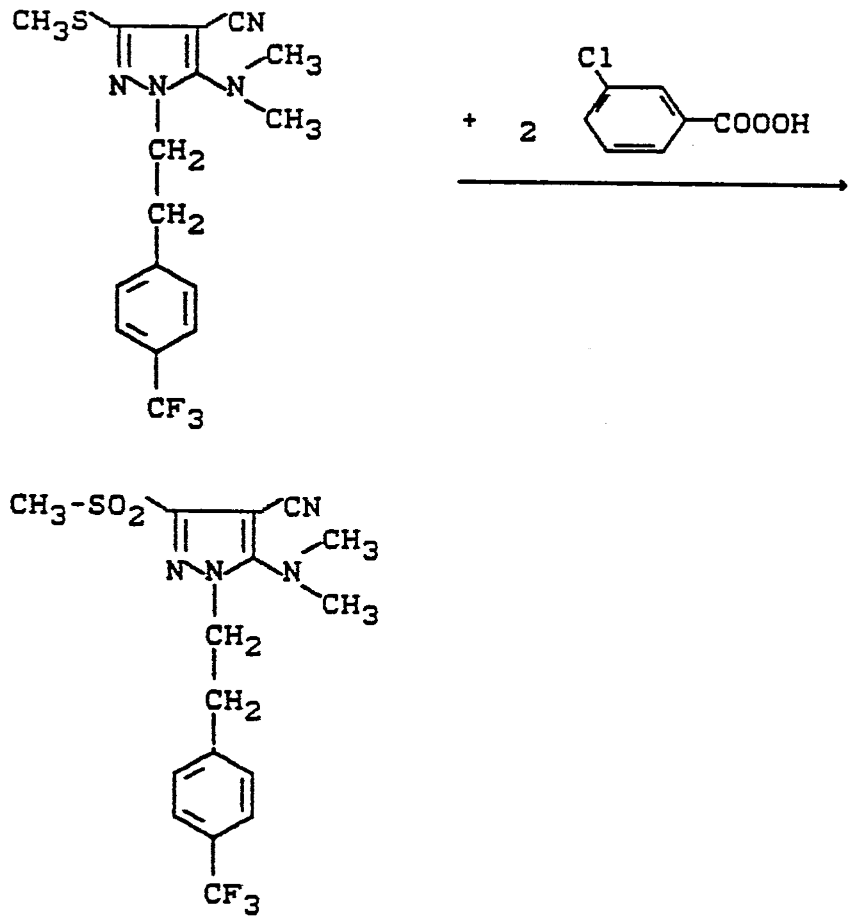

Die zur Durchführung des erfindungsgemäßen Verfahrens (a) als Ausgangsstoffe benötigten Aralkylhydrazine sind durch die Formel (II) allgemein definiert. In dieser Formel (II) steht Ar vorzugsweise für diejenigen Reste, die bereits im Zusammenhang mit der Beschreibung der erfindungsgemäßen Stoffe der Formel (I) als bevorzugt für diesen Substituenten genannt wurden.

Die Aralkylhydrazine der Formel (II) sind allgemein bekannte Verbindungen der organischen Chemie. Die 1,1,-Dicyanethylen-Derivate der Formel (III) sind ebenfalls bekannt (siehe z.B. Chem. Ber. 95, 2861, 2871 (1962)).

Die Umsetzung gemäß Verfahrensvariante (a) wird vorzugsweise in organischen Lösungsmitteln insbesondere in Alkoholen, cyclischen Ethern (wie Tetrahydrofuran), aromatischen Kohlenwasserstoffen (wie Benzol und Toluol), offenkettigen Ethern (wie Diethylether) oder chlorierten Kohlenwasserstoffen wie Methylenchlorid oder Chloroform durchgeführt. Die Reaktionstemperaturen betragen vorzugsweise 0 bis 60° C, insbesondere 10 bis 30° C. Die Reaktionszeit beträgt im allgemeinen ca. 2 bis ca. 20 Stunden.

Im allgemeinen werden molare Mengen der Ausgangskomponenten (II) und (III) im Lösungsmittel zusammengegeben und man rührt bei Raumtemperatur und Normaldruck. Anschließend engt man vorzugsweise am Rotationsverdampfer ein und kristallisiert aus einem geeigneten Lösungsmittel um.

Bei der Reaktionsvariante (b) sind die Ausgangsverbindungen der Formel (IV) allgemein bekannte Verbindungen der organischen Chemie. Es kommen hier Carbonsäureester, Carbonsäuren und Carbonsäurehalogenide (insbesondere Carbonsäurechloride) in Frage.

Man setzt die beiden Komponenten (Ia) und (IV) in an sich üblicher Art und Weise um, vorzugsweise in einem organischen Lösungsmittel insbesondere in cyclischen Ethern, wie Tetrahydrofuran, halogenierten Kohlenwasserstoffen wie Methylenchlorid oder Chloroform, Säurenitrilen wie Acetonitril oder aromatischen Kohlenwasserstoffen wie Benzol oder Toluol. Man setzt vorzugsweise Basen wie organische Amine oder auch Pyridin zu. Die Umsetzungstemperatur beträgt ca. 0 bis ca. 50° C, die Reaktionszeit beträgt ca. 5 bis ca. 40 Stunden. Vorzugsweise wird bei Normaldruck umgesetzt. Die Aufarbeitung erfolgt in üblicher, bereits bei Variante a) angegebener Art und Weise.

Bei der Reaktionsvariante (c) setzt man molare Mengen bis zum dreifachen Überschuß an an sich bekanntem Dialkylsulfat mit den Verbindungen der Formel (V) um. Die Umsetzung geschieht vorzugsweise in einem organischen Lösungsmittel, insbesondere in halogenierten Kohlenwasserstoffen wie Methylenchlorid oder Chloroform. Die Reaktionstemperaturen betragen von ca. 10° C bis ca. 40° C, die Reaktionszeit beträgt ca. 2 bis ca. 40 Stunden.

Es wurden hierbei äquivalente Mengen an Basen, insbesondere anorganischen Basen wie KOH, NaOH, $Na_2CO_3$ zugesetzt. Ferner werden gegebenenfalls Katalysatoren zur Reaktions beschleunigung eingesetzt. Als Katalysatoren kommen vorzugsweise Ammoniumsalze in Frage.

Die Umsetzung findet vorzugsweise im organischen Lösungsmittel unter Zusatz einer wäßrigen Lösung der Base sowie des Dialkylsulfats statt. Nach dem Rühren und Einengen wird aus einem geeigneten organischen Lösungsmittel umkristallisiert.

Bei der Reaktionsvariante (d) setzt man vorzugsweise ohne Lösungsmittel die Verbindung der Formel (VI) mit der molaren Menge bis zum 20-fachen Überschuß an Amin der Formel (VII) um. Die Umsetzungstemperatur beträgt ca. 0 bis ca. 50° C und die Reaktionszeit beträgt ca. 5 bis ca. 100 Stunden. Die Komponenten werden zusammengegeben, nach Abschluß der Reaktionszeit eingeengt und das Reaktionsprodukt der Formel (Id) wird vorzugsweise säulenchromatographisch abgetrennt.

Die bei der Herstellungsvariante (d) eingesetzten Ausgangsprodukte der Formel (VI) sind neu.

Sie sind wie folgt in guter Weise zugänglich:

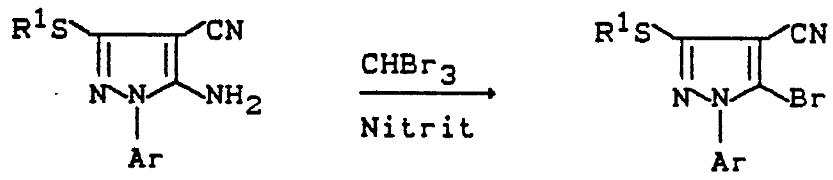


(Ia) (VI)

Bromoform dient hierbei gleichzeitig als Lösungsmittel. Als Nitrit wird vorzugsweise tert.-Butylnitrit, Isoamylnitrit oder Neopentylnitrit in jeweils molarer bis 1,5molarer Menge eingesetzt. Man rührt 1 bis 30 Stunden bei 10 bis 30° C, engt ein, nimmt in einem organischen Lösungsmittel vorzugsweise einen halogenierten Kohlenwasserstoff und insbesondere in Methylenchlorid auf, weäscht mit $NaHCO_3$-Lösung, trennt, trocknet und engt ein und kristallisiert gegebenenfalls aus einem geeigneten Lösungsmittel um.

Eine weitere Methode zur Herstellung von Ausgangsprodukten der Formel (VI) besteht darin, daß man das Pyrazol der Formel Ia wie folgt umsetzt:

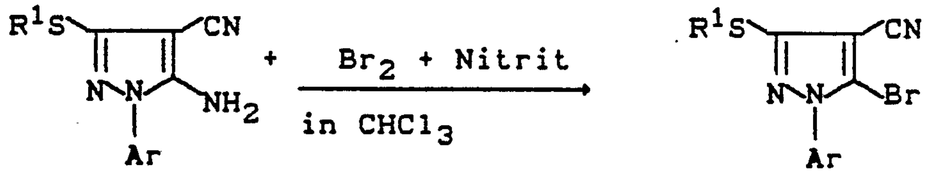


(Ia) (VI)

Hierbei dient als Lösungsmittel vorzugsweise ein halogenierter Kohlenwasserstoff wie Chloroform. Als Nitrite wurden die obengenannten eingesetzt. Man setzt an Nitrit und an Brom jeweils die ein-bis 1,7-molare Menge während ca. 10 bis ca. 30 Stunden bei einer Temperatur von ca. 10 bis ca. 50° C zu, engt ein, nimmt mit chloriertem Kohlenwasserstoff, insbesondere Methylenchlorid auf, schüttelt mit Bicarbonatlösung (insbesondere Natriumbicarbonatlösung aus) trocknet die organische Phase, engt ein und kristallisiert gegebenenfalls aus einem geeigneten organischen Lösungsmittel um.

Bei der Reaktionsvariante (e) setzt man 1-Arylalkyl-4-cyan-pyrazole der Formel (Id) mit einem Oxidationsmittel, vorzugsweise mit einer Persäure und insbesondere mit 3-Chlor-perbenzoesäure um. Als Lösungsmittel wurden polare organische Lösungsmittel, insbesondere Acetonitril, Methylenchlorid oder Chloroform verwendet. Die Reaktionstemperatur beträgt vorzugsweise 0 bis 40°C, die Reaktionsdauer im allgemeinen 2 bis 25 Stunden. Zur Herstellung des Sulfoxids (n = 1) setzt man äquimolare Mengen des Oxidationsmittels, vorzugsweise der Persäure zu, für die Herstellung der Sulfone (n = 2) benötigt man einen mindestens 2-molaren Überschuß an Oxidationsmittel (vorzugsweise Persäure). Die Reaktionspartner werden unter den oben aufgeführten Reaktionsbedingungen zusammengegeben. Nach Ablauf der Reaktion

engt man ein, wäscht vorzugsweise mit einer wäßrigen Bicarbonatlösung (vorzugsweise Natriumbicarbonat), schüttelt mit einem polaren organischen Lösungsmittel (beispielsweise mit Methylenchlorid) aus, trocknet die organische Phase und engt ein. Aus der eingeengten Phase kann das Reaktionsprodukt nach üblichen Methoden (z.B. Kristallisation oder Destillation) noch weiter gereinigt werden.

Die Wirkstoffe eignen sich bei guter Pflanzenverträglichkeit und günstiger Warmblütertoxizität zur Bekämpfung von tierischen Schädlingen, insbesondere Spinnentiere, die in der Landwirtschaft, in Forsten, im Vorrats-und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularia.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp.., Earias insulana, Heliothis spp., Spodoptera exigua, Mamestra brassicae, Panolis flammea, Prodenia litura, Spodoptera spp., Trichoplusiani, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B.Anobium punctatum, Rhizopertha dominica, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phrobia spp., Pegomyia hyoscyami, Ceratitis capitata, Dacus oleae, Tipula paludosa.

Aus der Ordnung der Siphonaptera z.B. Xenopsylla cheopis, Ceratophyllus spp.,
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.,

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.,

Die erfindungsgemäßen Wirkstoffe weisen auch eine gute fungizide Wirkung gegen Pyricularia oryzae am Reis auf.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur-und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Diemthylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgut, Montmorillonit oder Diatomeenerde und synthetische Gesteinsmehle, wie hochdisperse Keiselsäure, Aluminiumoxid und Silikate; als feste Trägerstoffe für Granulate kommen in Frage; z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl. Kokosnußschalen, Maiskolben und Tabakstengel; als Emulgier-und/oder -schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-und Metallphthalicyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a..

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene-und Vorratsschädlinge zeichnen sich die Wirkstoffe durch eine hervorragende Residualwirkung auf Holz und Ton sowie durch eine gute Alkalistabilität auf gekälkten Unterlagen aus.

## Herstellungsbeispiele

### Beispiel 1

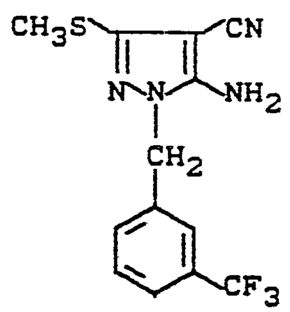

(Verfahren a)

8,5 g 1,1-Dicyano-2,2-bis-(methylmercapto)-ethylen und 9,5 g 3-Trifluormethyl-benzylhydrazin werden in 100 ml Ethanol zusammengegeben. Man rührt 15 Stunden lang bei 20° C und verdampft das Lösungsmittel am Rotationsverdampfer. Der Rückstand wird im Petrolether verrieben. Man erhält 12,4 g (80 % der Theorie) 1-(3-Trifluormethylbenzyl)-3-methylmercapto-4-cyano-5-amino-pyrazol vom Schmelzpunkt 142° C.

### Beispiel 2

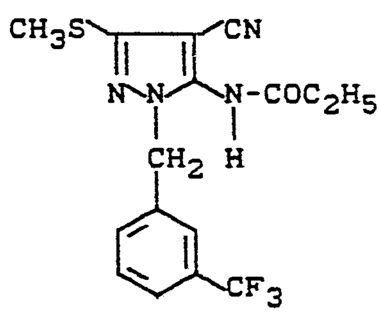

(Verfahren b)

3,2 g 1-(3-Trifluormethylbenzyl)-3-methylmercapto-4-cyan-5-amino-pyrazol und 2 g Propionsäurechlorid werden in 50 ml Chloroform gelöst, auf 0°C abgekühlt und mit 1 ml Pyridin versetzt. Nach 20-stündigem Rühren bei 20°C wird zum Chloroform 50 ml Ethanol zugegeben, mit 15 ml Ammoniumhydroxid-Lösung (25 %ig) alkalisch gestellt, bis zur Trockne eingeengt, in Chloroform aufgenommen, mit verdünnter Salzsäure und mit Wasser gewaschen, die Chloroformphase mit Natriumsulfat getrocknet und die organische Phase am Rotationsverdampfer eingeengt. Ausbeute: 2,8 g 1-(3-Trifluormethylbenzyl)-3-methylmercapto-4-cyano-5-propionylamino-pyrazol (76 % der Theorie), Schmelzpunkt: 181°C.

Beispiel 3

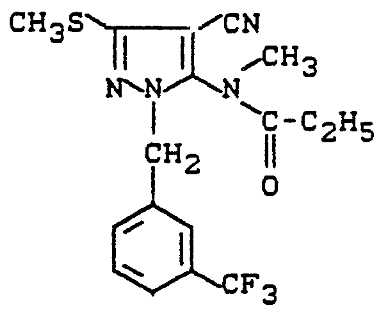

(Verfahren c)

Zu einer Lösung aus 3,7 g 1-(3-Trifluormethylbenzyl)-3-methylmercapto-4-cyan-5-propionylaminopyrazol in 50 ml Chloroform und 0,1 g Tributylbenzylammoniumchlorid sowie 1,3 g Dimethylsulfat, gibt man 50 ml einer 40 %igen wäßrigen Kaliumhydroxidlösung. Nach 72 Stunden Rühren bei 20°C wird die organische Phase abgetrennt und mit Wasser gewaschen, mit wasserfreiem Natriumsulfat getrocknet und eingeengt. Man erhält 3,4 g (90 % der Theorie) 1-(3-Trifluormethylbenzyl)-3-methyl-mercapto-4-cyan-5-N-methyl-N-propionyl-amino-pyrazol als ölige Substanz.

NMR-Spektraldaten: 3H 3,1 (s)
2H 1,5-2,1 (m)
3H 0,9 (t)

Beispiel 4

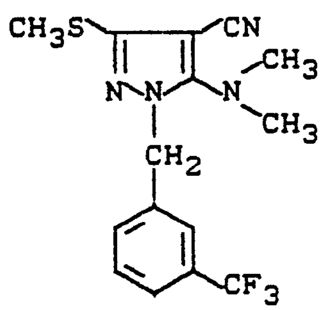

(Verfahren d)

Zu 3,8 g 1-(3-Trifluormethylbenzyl)-3-methylmercapto-4-cyan-5-brompyrazol werden 10 ml Dimethylamin gegeben und 72 Stunden lang bei 20°C gerührt. Überschüssiges Amin wird im Rotationsverdampfer entfernt und das rohe Endprodukt über eine Kieselgel-Säule gereinigt (Laufmittel: Gemisch Ethylether/Cyclohexan 2:1)).

Man erhält 3,0 g (88 % der Theorie) 1-(3-Trifluormethylbenzyl)-3-methylmercapto-4-cyan-5-dimethylamino-pyrazol.

Beispiel 4a

Herstellung des Vorprodukts

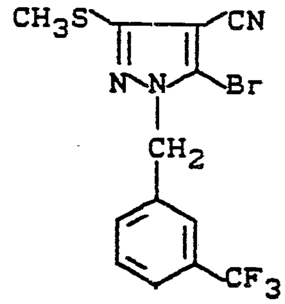

6,7 g 1-(3-Trifluormethylbenzyl)-3-methylmercapto-4-cyan-5-aminopyrazol sowie 2,3 ml tert.-Butylnitrit werden in 50 ml Bromoform 1 Stunde lang gerührt. Überschüssiges Bromoform wird abdestilliert, der Rückstand wird mit 50 ml Chloroform aufgenommen, mit Natriumbicarbonatlösung gewaschen, die organische Phase mit wasserfreiem Natriumsulfat getrocknet und anschließend wird am Rotationsverdampfer eingeengt.
Man erhält 8,2 g (praktisch quantitative Ausbeute) 1-(3-Trifluormethylbenzyl-3-methylmercapto-4-cyan-5-brompyrazol als ölige Substanz ($^1$H-NMR: $CH_3S$: 2,6 ppm (s)).

Beispiel 5

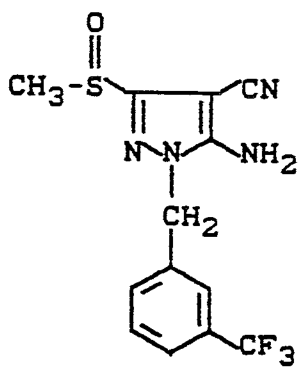

(Verfahren e)

2,7 g 1-(3-Trifluormethylbenzyl)-3-methylmercapto-4-cyan-5-amino-pyrazol werden in 30 ml Chloroform gelöst. Zu dieser Lösung tropft man eine Mischung aus 1,8 g 3-Chlorperbenzoesäure und 20 ml Chloroform bei 20°C zu und rührt 15 Stunden nach. Anschließend wird mit gesättigter Natriumhydrogencarbonat-Lösung, 2 %iger Natriumthiosulfat-Lösung und nochmals mit gesättigter wäßriger Natriumhydrogencarbonat-Lösung gewaschen. Die organische Phase wird mit wasserfreiem Natriumsulfat getrocknet, eingeengt und in Petrolether verrieben. Man erhält 2,2 g (75 % der Theorie) 1-(3-Trifluormethylbenzyl)-3-methylsulfinyl-4-cyan-5-amino-pyrazol vom Schmelzpunkt 143°C.

Beispiel 6

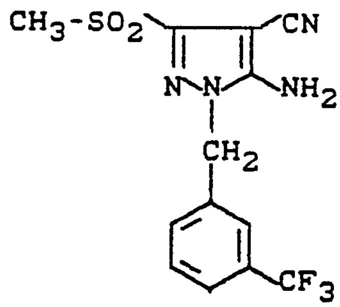

(Verfahren f)

2,7 g 1-(3-Trifluormethylbenzyl)-3-methylmercapto-4-cyan-5-aminopyrazol werden in 30 ml Chloroform gelöst. Zu dieser Lösung werden unter Rühren 3,6 g in 20 ml Chloroform gelöste 3-Chlor-perbenzoesäure bei 20°C zugetropft. Man rührt 15 Stunden nach. Anschließen wird mit gesättigter Natriumhydrogencarbonat-Lösung, 2 %iger Natriumthiosulfat-Lösung und nochmals mit gesättigter wäßriger Natriumhydrogencarbonatlösung gewaschen. Die organische Phase wird mit wasserfreiem Natriumsulfat getrocknet, eingeengt und im Petrolether verrieben. Man erhält 2,9 g (97 % der Theorie) 1-(3-Trifluormethylbenzyl)-3-methylsulfonyl-4-cyan-5-aminopyrazol vom Schmelzpunkt 110°C.

In Analogie zu den vorgenannten Beispielen lassen sich u.a. herstellen:

Allgemeine Formel

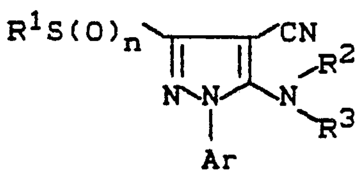

(I)

| Beispiel Nr. | Herstellungs-variante | $R^1$ | n | Ar | $R^2$ | $R^3$ | physikal. Konstante |
|---|---|---|---|---|---|---|---|
| 7 | b | $-CH_3$ | 0 | 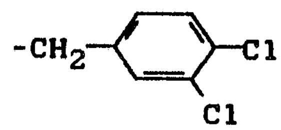 | H | 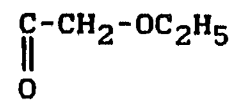 | Fp: 92° C |
| 8 | a | $-C_2H_5$ | 0 | 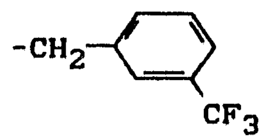 | H | H | Fp: 101° C |
| 9 | a | 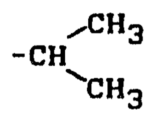 | 0 | 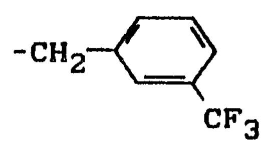 | H | H | Fp: 108° C |
| 10 | a | $-CH_3$ | 0 | 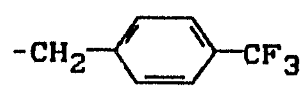 | H | H | Fp. 122° C |
| 11 | c | $-CH_3$ | 0 | 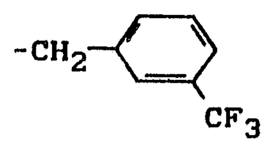 | H | $CH_3$ | Fp. 159° C |

| Beispiel Nr. | Herstellungs-variante | $R^1$ | n | Ar | $R^2$ | $R^3$ | physikal. Konstante |
|---|---|---|---|---|---|---|---|
| 12 | b | $-CH_3$ | 0 | 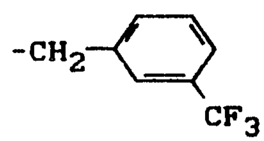 | H | 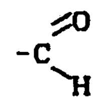 | Fp: 226° C |
| 13 | a | $-CH_3$ | 0 | 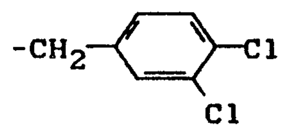 | H | H | Fp: 145° C |
| 14 | d | $-CH_3$ | 0 | 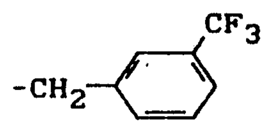 | -CH=CH-CH=CH- | | Fp: 64° C |
| 15 | c | $-CH_3$ | 0 | 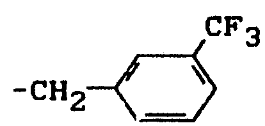 | $-COCH_3$ | $CH_3$ | $^1H$-NMR($CDCl_3$) 3,1 ppm(s) für $N-CH_3$ 2,2 ppm(s) für $-CO-CH_3$ |
| 16 | f | $-CH_3$ | 2 | 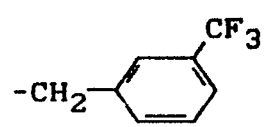 | $CH_3$ | $CH_3$ | Fp: 125° C |

| Beispiel Nr. | Herstellungs-variante | $R^1$ | n | Ar | $R^2$ | $R^3$ | physikal. Konstante |
|---|---|---|---|---|---|---|---|
| 17 | b | $-CH_3$ | 0 | 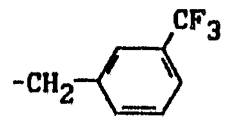 | $-CO-(CH_2)_3-$ | | Fp: 92° C |
| 18 | f | $-CH_3$ | 2 | 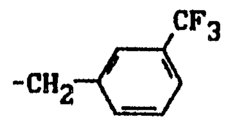 | $-CO-(CH_2)_3-$ | | Fp: 118° C |
| 19 | d | $-CH_3$ | 0 | 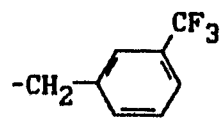 | $CH_3$ | $C_3H_7$-n | $^1$H-NMR($CDCl_3$)<br>2,8 ppm(s) für $N-CH_3$<br>0,8 ppm(t) für $-CH_2-CH_2-CH_3$<br>1,5 ppm(m) für $-CH_2-CH_2-CH_3$<br>3,0 ppm(dd) für $-CH_2-CH_2-CH_3$ |
| 20 | d | $CH_3$ | 0 | 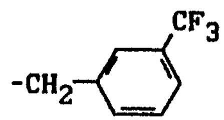 | $CH_3$ | $C_2H_5$ | $^1$H-NMR($CDCl_3$)<br>2,75 ppm(s) für $N-CH_3$<br>1,0 ppm(t) für $-CH_2-CH_3$<br>3,0 ppm(g) für $-CH_2-CH_3$ |
| 21 | d | $CH_3$ | 0 | 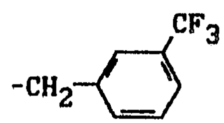 | 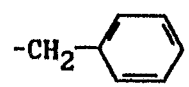 | $CH_3$ | $^1$-NMR($CDCl_3$)<br>2,75 ppm(s) für $N-CH_3$<br>4,2 ppm(s) für 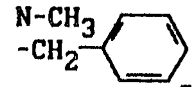<br>5,2 ppm(s) für 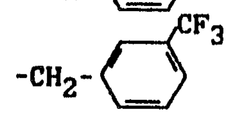 |

Beispiel A

Tetranychus-Test (resistent)

Lösungsmittel: 7 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: Verbindungen aus den Beispielen Nr. 1, 8, 13, 4, 3, 14, 5, 6, 15, 16, 19, 20.

## Ansprüche

1. 1-Aralkylpyrazole der Formel (I)

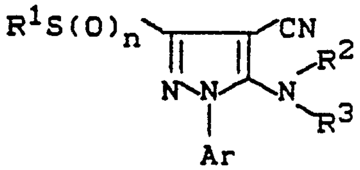

(I)

in welcher
Ar für gegebenenfalls substituiertes Aralkyl steht,
$R^1$ für Alkyl steht,
$R^2$ für Wasserstoff, Alkyl, Halogenalkyl, gegebenenfalls substituiertes Benzyl oder für die Reste $-COR^4$, $-COOR^4$, $-NR^5R^6$, $-CONR^5R^6$ steht,
wobei
$R^4$ für Wasserstoff, Alkyl, Halogenalkyl, Alkoxyalkyl und
$R^5$ und $R^6$ für Wasserstoff und/oder Alkyl steht,
$R^3$ für Wasserstoff oder Alkyl steht oder
$R^2$ und $R^3$ gemeinsam für die Gruppierungen $-CO-(CH_2)_m-$, $-(CH_2)_{\overline{m}}$ oder $-CH=CH-CH=CH-$stehen, wobei
m für die Zahlen 2, 3, 4 oder 5 steht und
n für die Zahlen 0, 1 oder 2 steht

2. 1-Arylpyrazole der Formel (I) gemäß Anspruch 1, dadurch gekennzeichnet, daß
Ar für gegebenenfalls einfach bis dreifach, gleich oder verschieden substituiertes Phenylalkyl mit 1 bis 4 Kohlenstoffatomen im geradkettigen oder verzweigten Alkylteil steht, wobei als Substituenten des Phenylrestes genannt seien: Halogen, Halogenalkyl oder Halogenalkoxy mit jeweils vorzugsweise 1 oder 2 Kohlenstoffatomen und 1 bis 5 gleichen oder verschiedenen Halogenatomen,
$R^1$ für Alkyl mit 1 bis 8 Kohlenstoffatomen steht,
$R^2$ für Wasserstoff, $C_1$-$C_8$-Alkyl, Halogenalkyl mit 1 oder 2 Kohlenstoffatomen und 1 bis 5 gleichen oder verschiedenen Halogenatomen oder für gegebenenfalls einfach bis dreifach, gleich oder verschieden substituiertes Benzyl steht, wobei als Phenylsubstituenten vorzugsweise genannt seien: Halogen, Alkyl mit 1 bis 4 Kohlenstoffatomen und Halogenalkyl mit 1 bis 2 Kohlenstoffatomen und 1 bis 5 gleichen oder verschiedenen Halogenatomen, sowie für die Reste
$-COR^4$, $-COOR^4$, $-NR^5R^6$, $-CONR^5R^6$ steht, wobei
$R^4$ für Wasserstoff, $C_1$-$C_8$-Alkyl, Halogenalkyl mit 1 oder 2 Kohlenstoffatomen und 1 bis 5 gleichen oder verschiedenen Halogenatomen oder Alkoxyalkyl mit 1 bis 4 Kohlenstoffatomen in jedem Alkylteil steht
und wobei
$R^5$ und $R^6$ für Wasserstoff oder $C_1$-$C_4$-Alkyl stehen,

$R^3$ für Wasserstoff oder $C_1$-$C_8$-Alkyl steht, oder
$R^2$ und $R^3$ gemeinsam für die Gruppierungen $-CO-(CH_2)_m$--$(CH_2)_m$-oder -CH = CH-CH = CH-stehen, wobei
m für die Zahlen 2, 3, 4 oder 5 steht und
n für die Zahlen 0, 1 oder 2 steht.

3. 1-Aralkylpyrazole der Formel (I) gemäß Anspruch 1, dadurch gekennzeichnet, daß
Ar für jeweils gegebenenfalls einfach oder zweifach, gleich oder verschieden durch Chlor oder Trifluormethyl substituiertes Benzyl, 1-Phenylethyl oder 2-Phenylethyl steht,
$R^1$ für Alkyl mit 1 bis 4 Kohlenstoffatomen steht,
$R^2$ für Wasserstoff, $C_1$-$C_4$-Alkyl oder für gegebenenfalls einfach oder zweifach, gleich oder verschieden substituiertes Benzyl steht, wobei als Phenylsubstituenten genannt seien: Fluor, Chlor, Brom, Methyl, Ethyl, Isopropyl und Trifluormethyl; sowie für die Reste $-NR^5R^6$, $-COR^4$, $-COOR^4$ und $-CONR^5R^6$ steht, wobei $R^4$ für Wasserstoff, $C_1$-$C_4$-Alkyl oder Alkoxyalkyl mit 1 bis 2 Kohlenstoffatomen in jedem Alkylteil steht und
$R^5$ und $R^6$ für Wasserstoff oder $C_1$-$C_4$-Alkyl stehen
$R^3$ für Wasserstoff oder $C_1$-$C_4$-Alkyl steht oder
$R^2$ und $R^3$ gemeinsam für die Gruppierungen $-CO-(CH_2)_m$--$(CH_2)_m$-oder -CH = CH-CH = CH-stehen, wobei
m für die Zahlen 2, 3, 4 oder 5 steht und
n für die Zahlen 0, 1 oder 2 steht.

4. Verfahren zur Herstellung von 1-Aralkylpyrazolen der Formel (I)

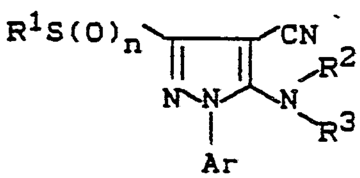

(I)

in welcher
Ar für gegebenenfalls substituiertes Aralkyl steht,
$R^1$ für Alkyl steht,
$R^2$ für Wasserstoff, Alkyl, Halogenalkyl, gegebenenfalls substituiertes Benzyl, oder für die Reste $-COR^4$, $-COOR^4$, $-NR^5R^6$, $-CONR^5R^6$ steht,
wobei
$R^4$ für Wasserstoff, Alkyl, Halogenalkyl, Alkoxyalkyl und
$R^5$ und $R^6$ für Wasserstoff und/oder Alkyl steht,
$R^3$ für Wasserstoff oder Alkyl steht oder
$R^2$ und $R^3$ gemeinsam für die Gruppierungen $-CO-(CH_2)_m$-, $-(CH_2)m$ oder -CH = CH-CH = CH-stehen, wobei
m für die Zahlen 2, 3, 4 oder 5 steht und
n für die Zahlen 0, 1 oder 2 steht,
dadurch gekennzeichnet, daß man

(a) zum Erhalt von Verbindungen der Formel (Ia)

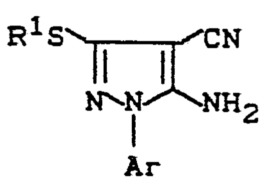

(Ia)

in welcher
$R^1$ und Ar die unter Formel (I) gegebene Bedeutung besitzen,
Aralkylhydrazine der Formel (II)
$Ar-NH-NH_2$ (II)
in welcher
Ar die oben angegebene Bedeutung besitzt
oder deren Säureadditionssalze mit 1,1-Dicyanethylen-Derivaten der Formel (III)

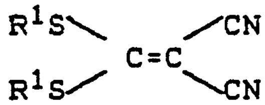

(III)

in welcher
$R^1$ die oben angegebene Bedeutung besitzt, gegebenenfalls in Gegenwart eines Lösungsmittels umsetzt, oder daß man

(b) zum Erhalt von Verbindungen der Formel (Ib)

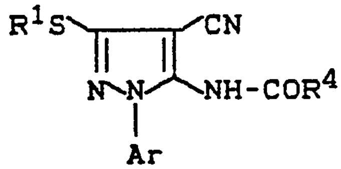

(Ib)

in welcher
Ar, $R^1$ und $R^4$ die unter Formel (I) angegebene Bedeutung besitzen, 1-Arylalkyl-4-cyan-5-amino-pyrazole der Formel (Ia)

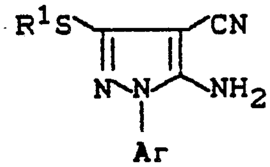

(Ia)

in welcher
Ar und $R^1$ die oben angegebene Bedeutung besitzen
mit Verbindungen der Formel
X-CO-$R^4$ (IV)
wobei
X = aktivierte Abgangsgruppe (wie Alkoxy, OH, Halogen) umsetzt, oder daß man

(c) zum Erhalt von Verbindungen der Formel (Ic)

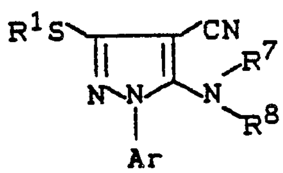

(Ic)

worin
Ar, $R^1$ die oben angegebene Bedeutung haben,
$R^7$ für Wasserstoff oder -CO$R^4$ steht und
$R^8$ für Alkyl steht,
Verbindungen der Formel (V)

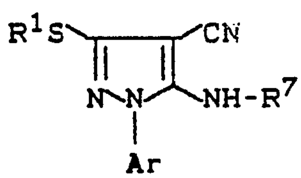

(V)

in welcher

Ar, $R^1$ und $R^7$ die oben angegebene Bedeutung besitzen, mit Dialkylsulfat umsetzt, oder daß man

(d) zum Erhalt von Verbindungen der Formel (Id)

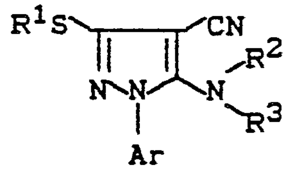

(Id)

in welcher

Ar, $R^1$, $R^2$ und $R^3$ die unter Formel (I) angegebene Bedeutung besitzen, 1-Arylalkyl-4-cyan-5-brom-pyrazole der Formel

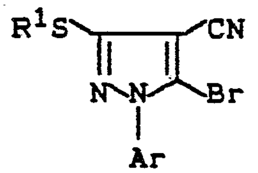

(VI)

in welcher

Ar und $R^1$ die oben angegebene Bedeutung besitzen, mit Aminen der Formel

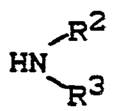

(VII)

umsetzt, oder daß man

(e) zum Erhalt von Verbindungen der Formel (Ie)

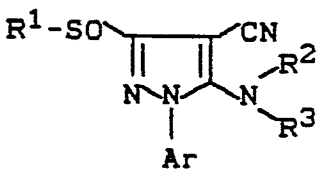

(Ie)

in welcher

Ar, $R^1$, $R^2$ und $R^3$ die oben angegebene Bedeutung besitzen, 1-Arylalkyl-4-cyan-pyrazole der Formel (Id)

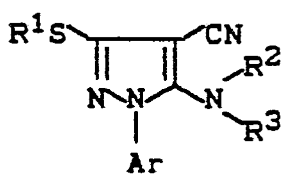

(Id)

in welcher

Ar, $R^1$, $R^2$ und $R^3$ die oben angegebene Bedeutung haben, mit äquimolaren Mengen eines Oxidationsmittels umsetzt, oder daß man

(f) zum Erhalt von Verbindungen der Formel (If)

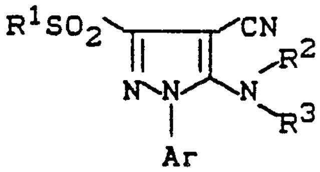

(If)

in welcher
Ar, $R^1$, $R^2$ und $R^3$ die oben angegebene Bedeutung haben,
1-Arylalkyl-4-cyan-pyrazole der Formel (Id)

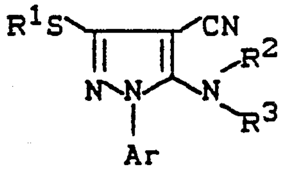

(Id)

mit mindestens der zweifach molaren Menge eines Oxidationsmittels umsetzt.

5. Schädlingsbekämpfungsmittel, gekennzeichnet durch einen Gehalt an mindestens einem 1-Aralkylpyrazol der Formel (I).

6. Akarizide Mittel, gekennzeichnet durch einen Gehalt an mindestens einem 1-Aralkylpyrazol der Formel (I).

7. Verfahren zur Bekämpfung von Spinnentieren, dadurch gekennzeichnet, daß man 1-Aralkylpyrazole der Formel (I) auf Spinnentiere und/oder deren Lebensraum einwirken läßt.

8. Verwendung von 1-Aralkylpyrazolen der Formel (I) zur Bekämpfung von Spinnentieren.

9. Verfahren zur Herstellung von Schädlingsbekämpfungsmitteln, dadurch gekennzeichnet, daß man 1-Aralkylpyrazole der Formel (I) mit Streckmitteln und/oder oberflächenaktiven Mitteln vermischt.